# EUROPEAN PATENT APPLICATION

(11) **EP 4 378 526 A1**
(43) Date of publication of application: **05.06.2024**
(21) Application number: 22306768.7
(22) Date of filing: 30.11.2022
(51) Int. Cl.: A61P 25/00, C07D 487/04, A61K 31/5517

(54) **NON PEPTIDERGIC AGONISTS OF OXYTOCIN RECEPTOR**

(71) Applicant: Université de Strasbourg, 67000 Strasbourg (FR); Centre National de la Recherche Scientifique, 75016 Paris (FR)
(72) Inventor: HIBERT, Marcel, 67114 Eschau (FR)
(74) Representative: Gevers & Orès

(57) **Abstract**

The present invention concerns a non peptidergic agonist of oxytocin receptor of formula (I), a pharmaceutical composition comprising such a compound and a pharmaceutically acceptable vehicle, and notably said compound for the treatment of all known pathologies where oxytocin has a beneficial effect such as autism spectrum disorders, in particular for the treatment of social interaction impairments in autism spectrum disorders.

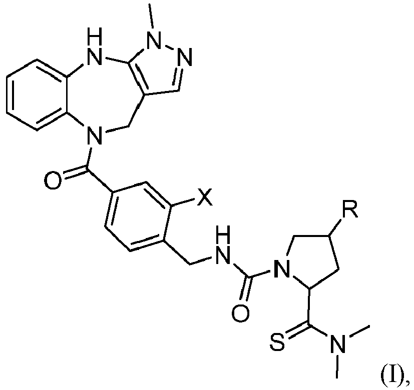

## Description

The present invention concerns a non peptidergic agonist of oxytocin receptor, a pharmaceutical composition comprising such a compound and a pharmaceutically acceptable vehicle, and notably said compound for the treatment of all known pathologies where oxytocin has a beneficial effect such as autism spectrum disorders, in particular for the treatment of social interaction impairments in autism spectrum disorders.

Millions of people worldwide suffer from some form of mental, neurological or behavioural disorders. Among them, many psychiatric disorders are characterized by a fundamental disruption of social behaviour. Common examples include autism spectrum disorder (ASD) and social anxiety disorder (SAD). Additionally, several disorders have social withdrawal as a secondary symptom; examples include depression, schizophrenia, major depressive disorder (MDD) and substance use disorders.

As described in the World Health Organization's International Classification of Diseases ICD-11, ASD is characterized by persistent deficits in the ability to initiate and to sustain reciprocal social interaction and social communication, and by a range of restricted, repetitive, and inflexible patterns of behaviour, interests or activities that are clearly atypical or excessive for the individual's age and sociocultural context. The onset of the disorder occurs during the developmental period, typically in early childhood, but symptoms may not become fully manifest until later, when social demands exceed limited capacities. Deficits are sufficiently severe to cause impairment in personal, family, social, educational, occupational or other important areas of functioning and are usually a pervasive feature of the individual's functioning observable in all settings, although they may vary according to social, educational, or other context. Individuals along the spectrum exhibit a full range of intellectual functioning and language abilities.

Approximately 1/100 children are diagnosed with autism spectrum disorder around the world, and prevalence estimates are increasing over time.

At present there are no approved drugs that target core symptoms of ASD, in particular disturbance of social interactions and communication, repetitive behaviors and restricted social interest. Currently prescribed drug-treatments in relation with these disorders (typically selective serotonin reuptake inhibitors (SSRIs), serotonin and noradrenaline reuptake inhibitors (SNRIs), stimulants and antipsychotics) are only targeting some associated symptoms (anxiety, depression, etc.) and have at best limited efficacy, poor compliance and vast side effect profiles, because they are indeed inefficient to address said core symptoms of ASD.

Researches have suggested that a promising target for treating these diseases is the neuropeptide oxytocin (OT). Animal studies showed that OT positively modulates a wide variety of social interactions, including maternal behaviour, courtship, sexual behaviour and peer-to-peer interaction. Results of human studies, in which OT is usually administered intranasally, showed that OT can increase trust and co-operation, improve social memory and decrease social fear. Moreover, recent clinical trials indicated that administration of OT to a person with autism and social anxiety can restore some aspects of social functioning. The OT receptor therefore has potential for drug discovery aimed at alleviating serious psychiatric disorders.

OT has also a beneficial effect on pathologies, diseases, conditions, disorders, such as pain, drug and/or alcohol addiction, depression, post-traumatic stress, anxiety, eating disorders, notably anorexia, bulimia and Prader-Willi syndrome, schizophrenia, neurodevelopmental disorders, social interaction disorders, borderline behaviour, ageing of the skin, muscles and bones.

However, OT is not suitable for a drug development, notably for CNS disorder, since OT is a big cyclic peptide (MW over 1000 Da) that does not survive primary metabolism, has a short physiological half-life (about 3 minutes half-life in blood) and does not readily penetrate the blood brain barrier.

Furthermore, OT is not specific since it binds, further to OT-receptors, to vasopressin 1 a receptor (VI aR). In fact, the OT receptors have a high degree of homology with the V1a receptors, meaning that selectivity can be extremely challenging.

Therefore, the development of a new compounds that overcome the above-mentioned drawbacks is strongly needed.

Accordingly, it is an object of the present invention to provide new compounds that target the core symptoms of ASD.

Another object of the present invention is to provide pharmaceutical compositions and compounds for their use to treat autism, autism spectrum disorders, in particular for the treatment of social interaction impairments in autism spectrum disorders, pain, drug and/or alcohol addiction, depression, post-traumatic stress, anxiety, eating disorders, notably anorexia, bulimia and Prader-Willi syndrome, schizophrenia, neurodevelopmental disorders, social interaction disorders, borderline behaviour, ageing of the skin, muscles, bones, erectile dysfunction, and any disease for which oxytocin is likely to have a beneficial effect.

Another object of the present invention is to provide dermatologic and/or cosmetic compositions, and methods to reduce ageing of the skin.

Another object of the present invention is to provide methods to facilitate erection, lactation, childbirth, breastfeeding, and/or post-partum period, comprising a step of administering to a subject in need thereof a compound as defined above.

Another object of the present invention is to provide compounds that have improved activity on endogenous OT and/or improved selectivity for endogenous OT, in particular over vasopressin V1a and/or V2 receptors.

Another object of the present invention is to provide compounds that are non-peptide small molecules.

Thus, in one aspect, the present invention relates to a compound of following formula (I): 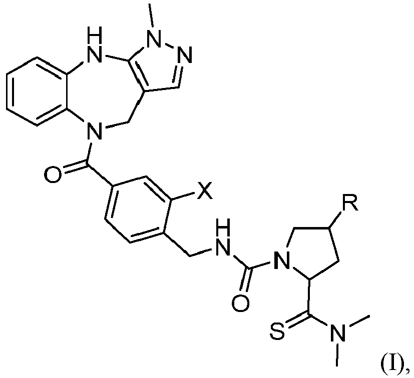 wherein:
X is chosen from -H, halogens, -(C₁-C₆)-alkyl groups, -O-(C₁-C₆)-alkyl groups and -O-CF₃, in particular -Me or -Cl, more particularly -Me;
R is chosen from the group comprising:
   - -(C₁-C₆)-alkyl groups, in particular -Me;
   - -O-(C₁-C₄)-alkyl groups, in particular -OMe;
   - -S-(C₁-C₄)-alkyl groups, in particular -SMe;
   - -O-(C₂-C₄)-alkyl-OH groups, in particular -O-(CH₂)ᵢ-OH, with i ranging from 2 to 4, i being more particularly 2;
   - -O(-(C₂-C₄)-alkyl)ₙ-OH groups, in particular -O(-CH₂-CH₂)ₙ-OH, with n ranging from 2 to 4, n being more particularly 2;
   - -O-(C₂-C₄)-alkyl-O-(C₂-C₄)-alkyl groups, in particular -O-(CH₂)ᵢ-OMe, with i ranging from 2 to 4, i being more particularly 2;
   - -O(-(C₂-C₄)-alkyl)ₘ-(C₂-C₄)-alkyl groups, in particular -O(-CH₂-CH₂)ₘ-OMe or -O(-CH₂-CH₂)ₘ-OEt, with m ranging from 2 to 4, m being more particularly 2;
   - -O-(C₁-C₄)-alkyl-Ar, in particular -O-(CH₂)ⱼ-Ar, with j ranging from 1 to 4, j being more particularly 1, and Ar being an aryl group being optionally substituted by at least one R" group, in particular at least one -OH group, said -O-(C₁-C₄)-alkyl-Ar group being for example of the following formula: 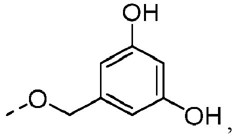 R" being chosen from the group comprising:
      ∘ -OH;
      ∘ a -O-(C₁-C₆)-alkyl or -O-cyclo-(C₃-C₆)-alkyl, in particular a -O-cyclopropyl;
      ∘ a -(C₁-C₆)-alkyl or -cyclo-(C₃-C₆)-alkyl, in particular a cyclopropyl;
      ∘ -(C₁-C₆)-alkyl-W groups, in particular -(CH₂)ₖ-W groups, said groups being optionally further substituted by at least a -W' group, with k ranging from 1 to 6, k being more particularly 1 or 2, and W and W' being ORₐ, with Rₐ representing -H or -(C₁-C₄)-alkyl, in particular H or Me, or a PEG group, in particular of following formula -(CH₂-CH₂-O)ₗ-H, with 1 ranging from 1 to 10, in particular from 2 to 4, for example 2; or W and W' being NR_{b}H, with R_{b} representing -H, a -(C₁-C₄)-alkyl group, or a -(C₁-C₄)-alkyl-Ar', with Ar' being an aryl or heteroaryl group, in particular a furanyl;
      ∘ -(C₁-C₆)-alkyl-Y-Z groups, in particular -(CH₂)ₗ-Y-Z, with 1 ranging from 1 to 6,1 being more particularly 1 or 2, Y being -O-, -NH- or being absent, and Z being chosen from saturated heterocyclic groups, in particular morpholinyl, tetrahydropyranyl, imidazolidinyl, said saturated heterocyclic groups being optionally substituted by at least one =O group, representing for example imidazolidine-2,4-dione; and heteroaryl groups, in particular tetrazolyl;
      ∘ An aryl or heteroaryl group, in particular a pyrazolyl, said group being optionally substituted by at least one R_{c} group chosen from -(C₁-C₆)-alkyl and -NH₂ groups;
      ∘ a -(C₀-C₆)-alkyl-C(=O)NH₂ or -(C₀-C₆)-alkyl-S(=O)₂NH₂ group, in particular a -C(=O)NH₂ or -(CH₂)₂-S(=O)₂NH₂ group;
   - Halogens, in particular -F;
   - -CF₃;
or R is -F and its geminal hydrogen is replaced by -F, R forming with the carbon atom C to which it is bound and its -F replaced geminal hydrogen a CF₂ residue;
or an enantiomer, diastereoisomer, and/or a pharmaceutically acceptable salt or solvate thereof.

The group -F₂ as used in the present invention, refers to two -F atoms bound to the same carbon atom C.

In a particular embodiment, R is chosen from -O-(C₁-C₄)-alkyl-Ar groups, in particular -O-(CH₂)ⱼ-Ar, with j ranging from 1 to 4, j being more particularly 1, and Ar being an aryl group being optionally substituted by at least one R" group as defined above.

In a particular embodiment, said Ar group is substituted by one R" group as defined above.

In another particular embodiment, said Ar group is substituted by two R" groups independently chosen as defined above.

In a particular embodiment, said Ar group is substituted by one -OH group and by one R" group as defined above.

In a more particular embodiment, said Ar group is chosen from: 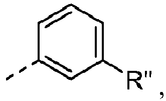 R" being as defined above, 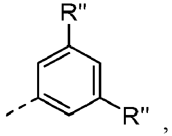 both R" groups being independently chosen as mentioned above, 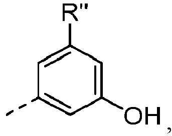 R' groups being chosen as mentioned above,

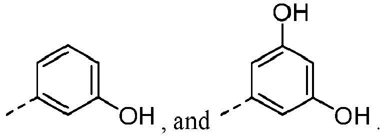

In a particular embodiment, R is chosen from: 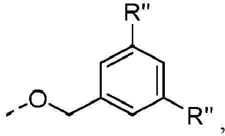 both R" groups being independently chosen as mentioned above, 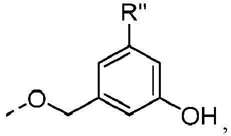 R" groups being chosen as mentioned above,

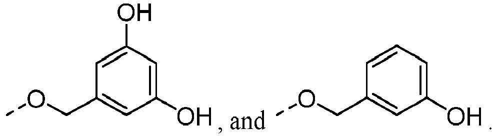

For the purpose of the invention, the term "pharmaceutically acceptable" is intended to mean what is useful to the preparation of a pharmaceutical composition, and what is generally safe and non-toxic, for a pharmaceutical use.

The term "pharmaceutically acceptable salt or solvate" is intended to mean, in the framework of the present invention, a salt or solvate of a compound which is pharmaceutically acceptable, as defined above, and which possesses the pharmacological activity of the corresponding compound.

The pharmaceutically acceptable salts comprise:
(1) acid addition salts formed with inorganic acids such as hydrochloric, hydrobromic, sulfuric, nitric and phosphoric acid and the like; or formed with organic acids such as acetic, benzenesulfonic, fumaric, glucoheptonic, gluconic, glutamic, glycolic, hydroxynaphtoic, 2-hydroxyethanesulfonic, lactic, maleic, malic, mandelic, methanesulfonic, muconic, 2-naphtalenesulfonic, propionic, succinic, dibenzoyl-L-tartaric, tartaric, p-toluenesulfonic, trimethylacetic, and trifluoroacetic acid and the like, and
(2) base addition salts formed when an acid proton present in the compound is either replaced by a metal ion, such as an alkali metal ion, an alkaline-earth metal ion, or an aluminium ion; or coordinated with an organic or inorganic base. Acceptable organic bases comprise diethanolamine, ethanolamine, N-methylglucamine, triethanolamine, tromethamine and the like. Acceptable inorganic bases comprise aluminium hydroxide, calcium hydroxide, potassium hydroxide, sodium carbonate and sodium hydroxide.

Acceptable solvates for the therapeutic use of the compounds of the present invention include conventional solvates such as those formed during the last step of the preparation of the compounds of the invention due to the presence of solvents. As an example, mention may be made of solvates due to the presence of water (these solvates are also called hydrates) or ethanol.

It is recognized that compounds of the present invention may exist in various stereoisomeric forms. As such, the compounds of the present invention include both diastereomers and enantiomers. The compounds are normally prepared as racemates and can conveniently be used as such, but individual enantiomers can be isolated or synthesized by conventional techniques if so desired. Such racemates and individual enantiomers and mixtures thereof form part of the present invention.

It is well known in the art how to prepare and isolate such optically active forms. Specific stereoisomers can be prepared by stereospecific synthesis using enantiomerically pure or enantiomerically enriched starting materials. The specific stereoisomers of either starting materials or products can be resolved and recovered by techniques known in the art, such as resolution of racemic forms, normal, reverse-phase, and chiral chromatography, recrystallization, enzymatic resolution, or fractional recrystallization of addition salts formed by reagents used for that purpose. Useful methods of resolving and recovering specific stereoisomers described in Eliel, E. L.; Wilen, S.H. Stereochemistry of Organic Compounds; Wiley: New York, 1994, and Jacques, J, et al. Enantiomers, Racemates, and Resolutions; Wiley: New York, 1981, each incorporated by reference herein in their entireties.

In a particular embodiment, the present invention relates to a compound as defined above, of following formula (I): 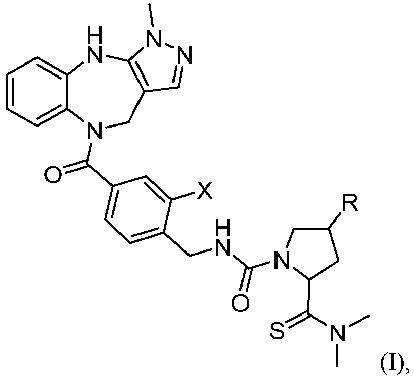 wherein:
X is chosen from -H, halogens, -(C₁-C₆)-alkyl groups, -O-(C₁-C₆)-alkyl groups and -O-CF₃, in particular -Me or -Cl, more particularly -Me;
R is chosen from the group comprising:
   - -O-(C₁-C₄)-alkyl groups, in particular -OMe;
   - -O-(C₂-C₄)-alkyl-OH groups, in particular -O-(CH₂)ᵢ-OH, with i ranging from 2 to 4, i being more particularly 2;
   - -O(-(C₂-C₄)-alkyl)ₙ-OH groups, in particular -O(-CH₂-CH₂)ₙ-OH, with n ranging from 2 to 4, n being more particularly 2;
   - -O-(C₂-C₄)-alkyl-O-(C₂-C₄)-alkyl groups, in particular -O-(CH₂)ᵢ-OMe, with i ranging from 2 to 4, i being more particularly 2;
   - -O(-(C₂-C₄)-alkyl)ₘ-(C₂-C₄)-alkyl groups, in particular -O(-CH₂-CH₂)ₘ-OMe or -O(-CH₂-CH₂)ₘ-OEt, with m ranging from 2 to 4, m being more particularly 2;
   - -O-(C₁-C₄)-alkyl-Ar, in particular -O-(CH₂)ⱼ-Ar, with j ranging from 1 to 4, j being more particularly 1, and Ar being an aryl group being optionally substituted by at least one R" group, in particular at least one -OH group, said -O-(C₁-C₄)-alkyl-Ar group being for example of the following formula: 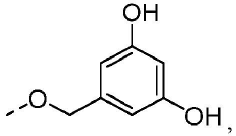 R" being chosen from the group comprising:
      ∘ -OH;
      ∘ a -O-(C₁-C₆)-alkyl or -O-cyclo-(C₃-C₆)-alkyl, in particular a -O-cyclopropyl;
      ∘ a -(C₁-C₆)-alkyl or -cyclo-(C₃-C₆)-alkyl, in particular a cyclopropyl;
      ∘ -(C₁-C₆)-alkyl-W groups, in particular -(CH₂)ₖ-W groups, said groups being optionally further substituted by at least a -W' group, with k ranging from 1 to 6, k being more particularly 1 or 2, and W and W' being ORₐ, with Rₐ representing -H or -(C₁-C₄)-alkyl, in particular H or Me, or a PEG group, in particular of following formula -(CH₂-CH₂-O)ₗ-H, with 1 ranging from 1 to 10, in particular from 2 to 4, for example 2; or W and W' being NR_{b}H, with R_{b} representing -H, a -(C₁-C₄)-alkyl group, or a -(C₁-C₄)-alkyl-Ar', with Ar' being an aryl or heteroaryl group, in particular a furanyl;
      ∘ -(C₁-C₆)-alkyl-Y-Z groups, in particular -(CH₂)ₗ-Y-Z, with 1 ranging from 1 to 6,1 being more particularly 1 or 2, Y being -O-, -NH- or being absent, and Z being chosen from saturated heterocyclic groups, in particular morpholinyl, tetrahydropyranyl, imidazolidinyl, said saturated heterocyclic groups being optionally substituted by at least one =O group, representing for example imidazolidine-2,4-dione; and heteroaryl groups, in particular tetrazolyl;
      ∘ An aryl or heteroaryl group, in particular a pyrazolyl, said group being optionally substituted by at least one R_{c} group chosen from -(C₁-C₆)-alkyl and -NH₂ groups;
      ∘ a -(C₀-C₆)-alkyl-C(=O)NH₂ or -(C₀-C₆)-alkyl-S(=O)₂NH₂ group, in particular a -C(=O)NH₂ or -(CH₂)₂-S(=O)₂NH₂ group;
   - Halogens, in particular -F.

In another particular embodiment, the present invention relates to a compound as defined above, of following formula (I): 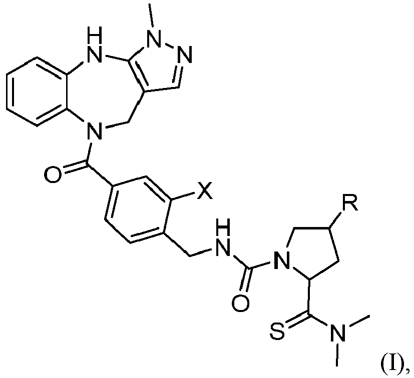 wherein:
X is chosen from -H, halogens, -(C₁-C₆)-alkyl groups, -O-(C₁-C₆)-alkyl groups and -O-CF₃, in particular -Me or -Cl, more particularly -Me;
R is chosen from -O-(C₁-C₄)-alkyl-Ar groups, in particular -O-(CH₂)ⱼ-Ar, with j ranging from 1 to 4, j being more particularly 1, and Ar being optionally an aryl group substituted by at least one R" group, in particular at least one -OH group, said -O-(C₁-C₄)-alkyl-Ar group being for example of the following formula: 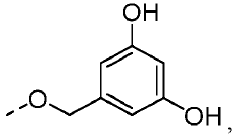 R" being chosen from the group comprising:
   ∘ -OH;
   ∘ a -O-(C₁-C₆)-alkyl or -O-cyclo-(C₃-C₆)-alkyl, in particular a -O-cyclopropyl;
   ∘ a -(C₁-C₆)-alkyl or -cyclo-(C₃-C₆)-alkyl, in particular a cyclopropyl;
   ∘ -(C₁-C₆)-alkyl-W groups, in particular -(CH₂)ₖ-W groups, said groups being optionally further substituted by at least a -W' group, with k ranging from 1 to 6, k being more particularly 1 or 2, and W and W' being ORₐ, with Rₐ representing -H or -(C₁-C₄)-alkyl, in particular H or Me, or a PEG group, in particular of following formula -(CH₂-CH₂-O)ₗ-H, with 1 ranging from 1 to 10, in particular from 2 to 4, for example 2; or W and W' being NR_{b}H, with R_{b} representing -H, a -(C₁-C₄)-alkyl group, or a -(C₁-C₄)-alkyl-Ar', with Ar' being an aryl or heteroaryl group, in particular a furanyl;
   ∘ -(C₁-C₆)-alkyl-Y-Z groups, in particular -(CH₂)ₗ-Y-Z, with 1 ranging from 1 to 6,1 being more particularly 1 or 2, Y being -O-, -NH- or being absent, and Z being chosen from saturated heterocyclic groups, in particular morpholinyl, tetrahydropyranyl, imidazolidinyl, said saturated heterocyclic groups being optionally substituted by at least one =O group, representing for example imidazolidine-2,4-dione; and heteroaryl groups, in particular tetrazolyl;
   ∘ An aryl or heteroaryl group, in particular a pyrazolyl, said group being optionally substituted by at least one R_{c} group chosen from -(C₁-C₆)-alkyl and -NH₂ groups;
   ∘ a -(C₀-C₆)-alkyl-C(=O)NH₂ or -(C₀-C₆)-alkyl-S(=O)₂NH₂ group, in particular a -C(=O)NH₂ or -(CH₂)₂-S(=O)₂NH₂ group.

In a particular embodiment, the present invention relates to a compound as defined above, of one of the following formulae: 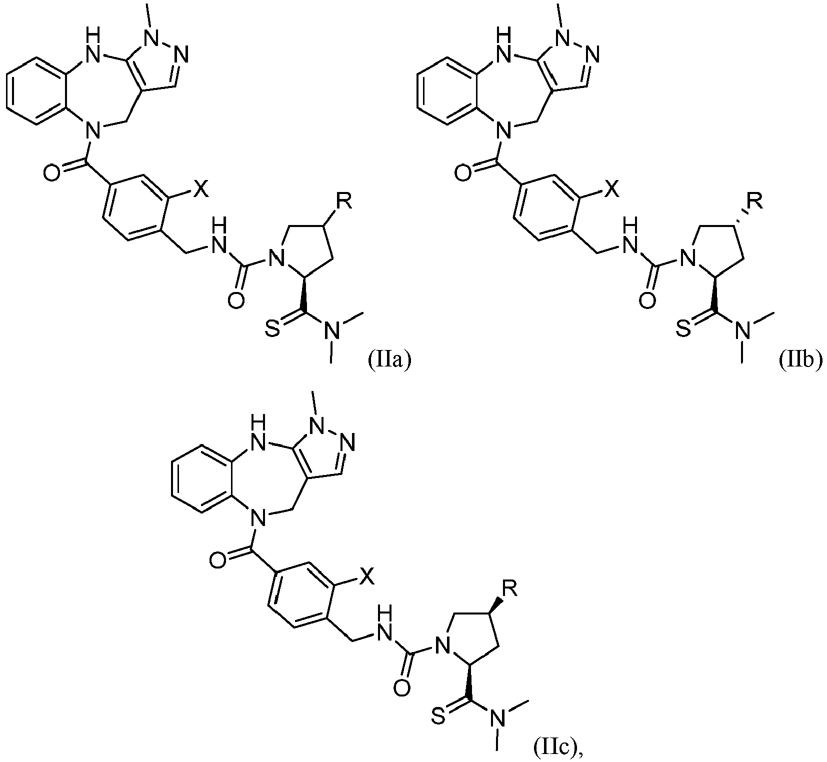 wherein X and R are as defined above.

In a particular embodiment, X is Me.

In another particular embodiment, X is Cl.

In a particular embodiment, R is not a -O-(C₁-C₄)-alkyl group, in particular not - OMe.

In a particular embodiment, the present invention relates to a compound as defined above, wherein R is chosen from:
-F,

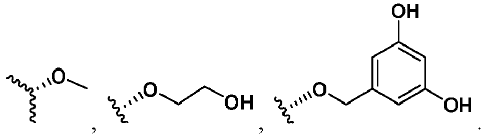

In another aspect, the present invention also relates to a pharmaceutical composition comprising a compound as defined above in admixture with at least one pharmaceutical acceptable excipient.

All the embodiments described above in relation with the compound of the invention apply here as well, alone or in any combination.

The compound or the pharmaceutical composition of the present invention may be administered in the form of a conventional pharmaceutical composition by any route including orally, intramuscularly, subcutaneously, topically, intranasally, intraperitoneally, intrathoracially, intravenously, epidurally, intrathecally, intracerebroventricularly and by injection into the joints, in particular orally, intravenously or intranasally.

The dosage will depend on the route of administration, the severity of the disease, age and weight of the patient and other factors normally considered by the attending physician, when determining the individual regimen and dosage level at the most appropriate for a particular patient.

For preparing pharmaceutical compositions from the compounds of the present invention, inert, pharmaceutically acceptable carriers can be either solid or liquid. Solid form preparations include powders, tablets, dispersible granules, capsules, cachets, and suppositories.

A solid carrier can be one or more substances, which may also act as diluents, flavouring agents, solubilizers, lubricants, suspending agents, binders, or tablet disintegrating agents; it can also be an encapsulating material.

Tablets, powders, cachets, and capsules can be used as solid dosage forms suitable for oral administration.

Liquid form compositions include solutions, suspensions, and emulsions. For example, sterile water or propylene glycol solutions of the active compounds may be liquid preparations suitable for parenteral administration. Liquid compositions can also be formulated in solution in aqueous polyethylene glycol solution.

Aqueous solutions for oral administration can be prepared by dissolving the active component in water and adding suitable colorants, flavouring agents, stabilizers, and thickening agents as desired. Aqueous solutions for oral use can be made by dispersing the finely divided active component in water together with a viscous material such as natural synthetic gums, resins, methyl cellulose, sodium carboxymethyl cellulose, and other suspending agents known to the pharmaceutical formulation art.

Compositions comprising microspheres based on polymers such as hyaluronic acid, dextran and/or starch can for example be used for intranasal administration.

Depending on the mode of administration, the pharmaceutical composition will according to one embodiment of the present invention include 0.05% to 99% weight (percent by weight), according to an alternative embodiment from 0.10 to 50% weight, of the compound of the present invention, all percentages by weight being based on total composition. A therapeutically effective amount for the practice of the present invention may be determined, by the use of known criteria including the age, weight and response of the individual patient, and interpreted within the context of the disease which is being treated or which is being prevented, by one of ordinary skills in the art.

In another aspect, the present invention also relates to a dermatologic and/or cosmetic composition comprising a compound as defined above in admixture with at least one pharmaceutical acceptable excipient.

All the embodiments described above in relation with the compound of the invention apply here as well, alone or in any combination.

The dermatologic and/or cosmetic composition of the present invention may be administered in the form of a conventional dermatologic and/or cosmetic composition by any route including orally, topically, in particular topically.

The dosage will depend on the route of administration, the severity of the disease, condition or disorder, age and weight of the patient and other factors normally considered by the attending physician or practitioner, when determining the individual regimen and dosage level at the most appropriate for a particular patient.

For preparing dermatologic and/or cosmetic compositions from the compounds of the present invention, inert, pharmaceutically acceptable carriers can be either solid or liquid.

The dermatologic and/or cosmetic comprising composition may be in any suitable form commonly used in cosmetics. In particular embodiments, the composition is selected from a solution, a suspension, a cream, a lotion, a paste, an emulsion, a gel, a foundation, a serum, and an ointment.

Depending on the mode of administration, the dermatologic and/or cosmetic composition will according to one embodiment of the present invention include 0.05% to 99% weight (percent by weight), according to an alternative embodiment from 0.10 to 50% weight, of the compound of the present invention, all percentages by weight being based on total composition.

In another aspect, the present invention also relates to a compound as defined above for use in the prevention and/or treatment of a disease, condition or disorder, which is autism, autism spectrum disorders, in particular for the treatment of social interaction impairments in autism spectrum disorders, pain, drug and/or alcohol addiction, depression, post-traumatic stress, anxiety, eating disorders, notably anorexia, bulimia and Prader-Willi syndrome, schizophrenia, neurodevelopmental disorders, social interaction disorders, borderline behaviour, ageing of the skin, muscles, bones, erectile dysfunction, and any disease for which oxytocin is known to have a beneficial effect.

All the embodiments described above in relation with the compound of the invention apply here as well, alone or in any combination.

In a particular embodiment, the present invention relates to a compound as defined above for use in the prevention and/or treatment of a disease, condition or disorder, which is autism, autism spectrum disorders, in particular for the treatment of social interaction impairments in autism spectrum disorders.

In another aspect, the present invention also relates to the use of a compound as defined above to relieve or reduce pain. All the embodiments described above in relation with the compound of the invention apply here as well, alone or in any combination.

In another aspect, the present invention also relates to the use of a compound as defined above to reduce ageing of the skin, muscles, bones, in particular of the skin.

All the embodiments described above in relation with the compound of the invention apply here as well, alone or in any combination.

In another aspect, the present invention also relates to the use of a compound as defined above to facilitate erection, lactation, childbirth, breastfeeding, and/or post-partum period.

All the embodiments described above in relation with the compound of the invention apply here as well, alone or in any combination.

In another aspect, the invention also relates to a method of prevention and/or treatment of a disease, condition or disorder, which is autism, autism spectrum disorders, in particular for the treatment of social interaction impairments in autism spectrum disorders, pain, drug and/or alcohol addiction, depression, post-traumatic stress, anxiety, eating disorders, notably anorexia, bulimia and Prader-Willi syndrome, schizophrenia, neurodevelopmental disorders, social interaction disorders, borderline behaviour, ageing of the skin, muscles, bones, erectile dysfunction, and any disease for which oxytocin is known to have a beneficial effect, comprising administering to a subject in need thereof a compound as defined above.

In another aspect, the invention also relates to a method to reduce ageing of the skin, muscles, bones, in particular of the skin, or to facilitate erection, lactation, childbirth, breastfeeding, and/or post-partum period, comprising administering to a subject in need thereof a compound as defined above.

In another aspect, the invention also relates to a composition comprising at least one compound as defined above and a diuretic, for example urea, or its salt, or a V2r antagonist, in particular a vaptan such as tolvaptan, lixivaptan, or satavaptan, as a combination product for simultaneous, separate or spread therapeutic use in the prevention and/or treatment of a disease, condition or disorder, which is autism, autism spectrum disorders, in particular for the treatment of social interaction impairments in autism spectrum disorders, pain, drug and/or alcohol addiction, depression, post-traumatic stress, anxiety, eating disorders, notably anorexia, bulimia and Prader-Willi syndrome, schizophrenia, neurodevelopmental disorders, social interaction disorders, borderline behaviour, ageing of the skin, muscles, bones, erectile dysfunction, and any disease for which oxytocin is known to have a beneficial effect. In another aspect, the invention also relates to a composition comprising at least one compound as defined above and a diuretic, for example urea, or its salt, or a V2r antagonist, in particular a vaptan such as tolvaptan, lixivaptan, or satavaptan, as a combination product for simultaneous, separate or spread therapeutic use to reduce ageing of the skin, muscles, bones, in particular of the skin, or to facilitate erection, lactation, childbirth, breastfeeding, and/or post-partum period.

### Definitions

The following terms and expressions contained herein are defined as follows:
As used herein, a range of values in the form "x-y" or "x to y", or "x through y", include integers x, y, and the integers therebetween. For example, the phrases "1-6", or "1 to 6" or "1 through 6" are intended to include the integers 1, 2, 3, 4, 5, and 6. Preferred embodiments include each individual integer in the range, as well as any subcombination of integers. For example, preferred integers for "1-6" can include 1, 2, 3, 4, 5, 6, 1-2, 1-3, 1-4, 1-5, 2-3, 2-4, 2-5, 2-6, etc.

The term "-(C₁-C₆)alkyl", as used in the present invention, refers to a straight or branched saturated hydrocarbon chain containing from 1 to 6 carbon atoms including, but not limited to, methyl, ethyl, n-propyl, iso-propyl, n-butyl, iso-butyl, sec-butyl, t-butyl, n-pentyl, n-hexyl, and the like.

The term "aryl", as used in the present invention, and which may be abbreviated "Ar", refers in particular to an aromatic hydrocarbon group comprising preferably 6 to 10 carbon atoms and comprising one or more, notably 1 or 2, fused rings, such as, for example, a phenyl or naphtyl group. Advantageously, it will be a phenyl group.

The term "-(C₁-C₄)-alkyl-aryl", as used in the present invention, refers to aryl group as defined above bound to the molecule via a (C₁-C₄)alkyl group as defined above.

The term "-(C₃-C₆)-cycloalkyl" as used in the present invention refers to a saturated hydrocarbon monocycle having 3-6 carbon atom members.

The term " saturated heterocyclic group" as used in the present invention refers in particular to a saturated hydrocarbon monocycle or polycycle (comprising fused, bridged or spiro rings), such as a bicycle, in which one or more, advantageously 1 to 4, and more advantageously 1 or 2, carbon atoms have each been replaced with a heteroatom selected from nitrogen, oxygen and sulphur atoms, and notably being a nitrogen atom. Advantageously, the heterocycle comprises 5 to 15, notably 5 to 10 atoms in the ring(s). The ring(s) of the heterocycle has/have advantageously 5 or 6 members.

According to a particular embodiment, the saturated heterocyclic group is in particular a saturated, hydrocarbon monocycle or bicycle (comprising fused, bridged or spiro rings, notably fused rings), each cycle having 5 or 6 members and 1 to 4, notably 1 or 2, carbon atoms having each been replaced with a nitrogen or oxygen atom, notably a nitrogen atom.

The term "heteroaryl" as used in the present invention refers in particular to an aromatic hydrocarbon monocycle or bicycle (i.e. comprising fused rings), each cycle having 5 or 6 members, notably 6 members, and 1 to 4, notably 1 or 2, carbon atoms having each been replaced with a nitrogen or oxygen atom, notably a nitrogen atom.

A heteroaryl can be notably thiophene, furan, pyrrole, imidazole, pyrazole, oxazole, isoxazole, thiazole, isothiazole, triazoles (1,2,3-triazole and 1,2,4-triazole), benzofuran, indole, benzothiophene, benzimidazole, indazole, benzoxazole, benzisoxazole, benzothiazole, benzisothiazole, pyridine, pyrimidine, pyridazine, pyrazine, triazine, quinoline, isoquinoline, quinoxaline, quinazoline, etc.

The term "-(C₁-C₄)alkyl-heteroaryl", as used in the present invention, refers to aryl group as defined above bound to the molecule via a (C₁-C₄)alkyl group as defined above.

The term "halogen", as used in the present invention, refers to a fluorine, bromine, chlorine or iodine atom.

### Synthesis

The compounds of the present invention may be prepared in a number of methods well known to those skilled in the art, including, but not limited to those described below, or through modifications of these methods by applying standard techniques known to those skilled in the art of organic synthesis. The appropriate modifications and substitutions will be readily apparent and well known or readily obtainable from the scientific literature to those skilled in the art. In particular, such methods can be found in R.C. Larock, Comprehensive Organic Transformations, Wiley-VCH Publishers, 2018.

All processes disclosed in association with the present invention are contemplated to be practiced on any scale, including milligram, gram, multigram, kilogram, multikilogram or commercial industrial scale.

It will be appreciated that the compounds of the present invention may contain one or more asymmetrically substituted carbon atoms, and may be isolated in optically active or racemic forms. Thus, all chiral, diastereomeric, racemic forms, isomeric forms of a structure are intended, unless the specific stereochemistry or isomeric form is specifically indicated. It is well-known in the art how to prepare and isolate such optically active forms. For example, mixtures of stereoisomers may be separated by standard techniques including, but not limited to, resolution of racemic forms, normal, reverse-phase, and chiral chromatography, preferential salt formation, recrystallization, and the like, or by chiral synthesis either from chiral starting materials or by deliberate synthesis of target chiral centers.

Compounds of the present invention may be prepared by a variety of synthetic routes. The reagents and starting materials are commercially available, or readily synthesized by well-known techniques by one of ordinary skill in the arts. All substituents, unless otherwise indicated, are as previously defined.

In the reactions described hereinafter, it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see T.W. Greene and P. G. M. Wuts in Protective Groups in Organic Chemistry, 3rd ed., John Wiley and Sons, 1999; J. F. W. McOmie in Protective Groups in Organic Chemistry, Plenum Press, 1973.

The reagents and starting materials are commercially available, or readily synthesized by well-known techniques by one of ordinary skill in the arts.

In particular, the compounds defined above are obtained according to the following procedure: 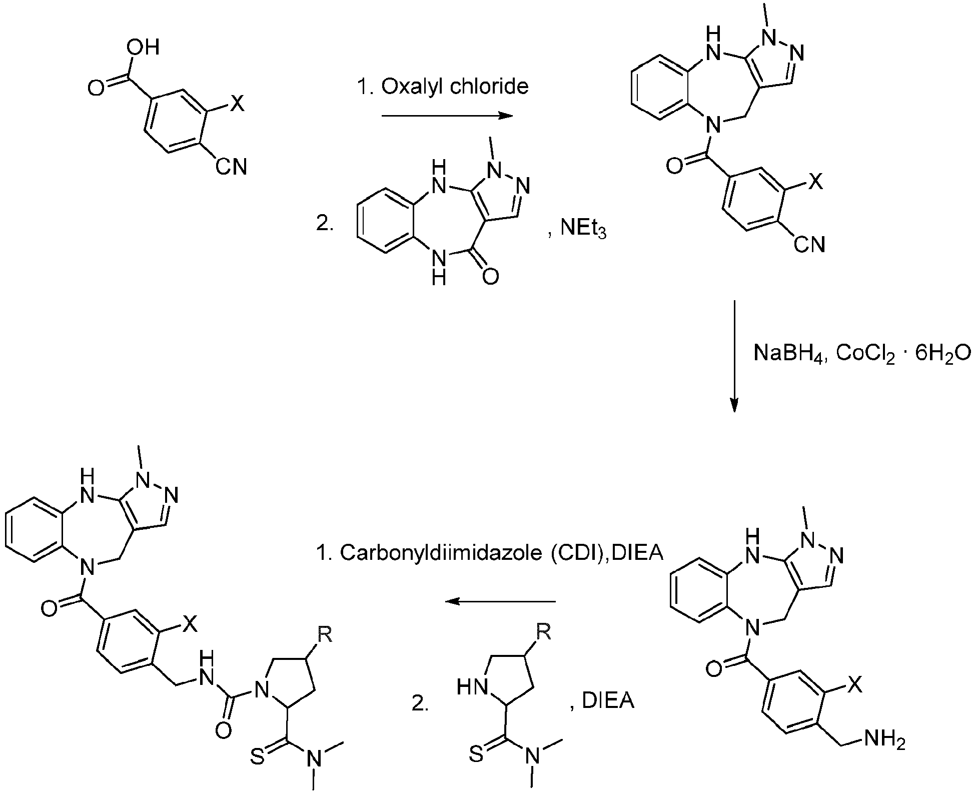 wherein X and R are as described above, or being protected by an ad hoc protecting group.

### EXAMPLES

### Example 1 : Synthesis of the compounds of the invention

Compounds of the invention have been obtained as follows: 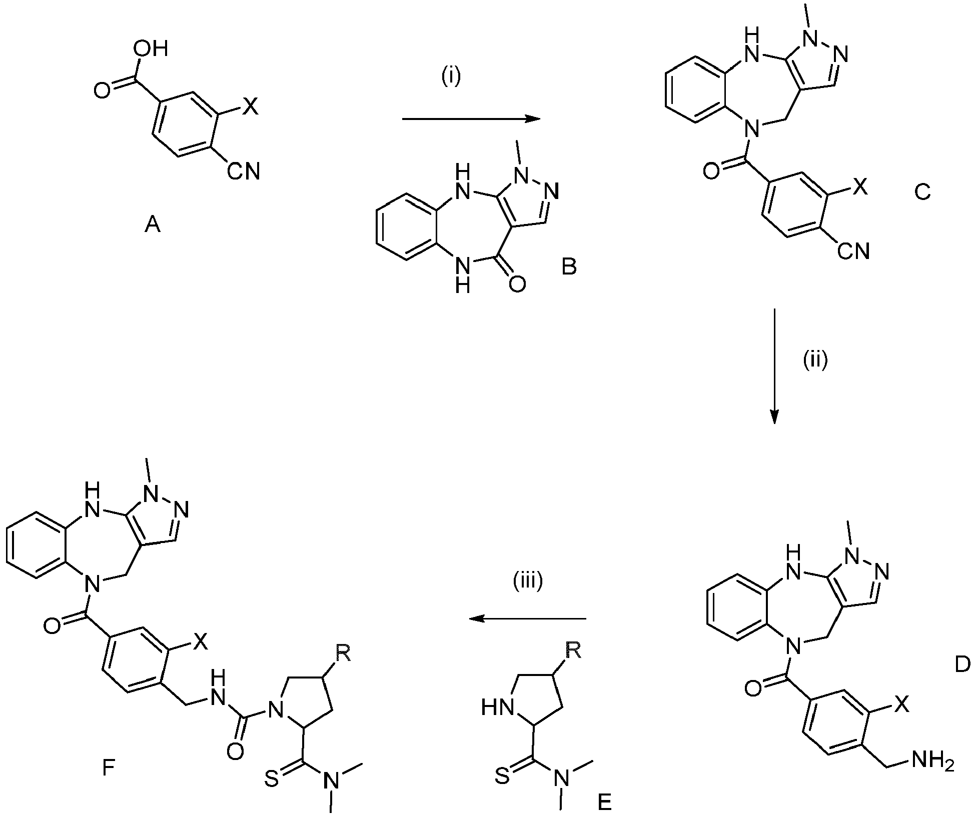 wherein X and R are as described above, or being protected by an ad hoc protecting group.

### Step (i):

To a solution of compound **A** (2 eq.) in anhydrous CH₂Cl₂, and DMF (a few drops), was added dropwise oxalyl chloride (4 eq.). The mixture was stirred for 15 minutes at 0°C, 2 hours at RT, concentrated under reduced pressure and dried under vacuum for 1 hour. The residue was dissolved in CH₂Cl₂, and added dropwise at 0°C to a solution of compound B (1 eq.) and triethylamine (2 eq.) in anhydrous CH₂Cl₂. The obtained mixture was stirred for 30 minutes at 0°C then overnight at RT. The crude product was dissolved in CH₂Cl₂ and the organic phase was washed with saturated KHSO4. The aqueous phase was extracted with a CHCl₃/PrOH 8/2 v/v mixture. The combined organic phases were washed with saturated NaHCO₃, dried over Na₂SO₄, and evaporated under reduced pressure. Purification by silica gel chromatography (CH₂Cl₂/MeOH, 95/5 v/v) gave product **C.**

### Step (ii):

To a solution of compound C (1 eq.) in anhydrous methanol at 0°C, were added portionwise over 10 minutes cobalt chloride hexahydrate (2 eq.), and sodium borohydride (10 eq.). The obtained black mixture was stirred for 10 minutes at 0°C, then 1 hour at RT. The mixture was neutralized to pH 7-8 with a 1M KHSO₄ solution. Methanol was evaporated under reduced pressure. The residue was dissolved in 1M KHSO₄. The formed precipitate was filtered and washed with diethyl ether. The aqueous phase was extracted with diethyl ether and then basified to pH>10 with a 10M NaOH solution. The aqueous phase was again extracted with diethyl ether. The combined organic phases were dried over Na₂SO₄ and evaporated under reduced pressure to yield compound **D.**

### Step (iii):

A solution of compound **D** (1 eq.), of carbonyldiimidazole (1.2 eq) and of DIEA (1.5 eq) in DMF was stirred for 3 hours at RT. To this mixture was added a solution of compound **E** and DIEA (2.5 eq) in DMF. The reactional mixture was stirred overnight at RT and then evaporated under reduced pressure. The residue was purified by semi-preparative HPLC to obtain expected product **F.**

### Example 2 : Biological assays

The activation constants (EC50 in nM) were measured in CHO cell lines expressing oxytocin receptor by IP-One IP₁ accumulation assay. These values are the mean ± SEM of at least three separate experiments performed in triplicate. The maximal stimulations (Eₘₐₓ) are expressed as percentages of the endogenous agonist maximal stimulation (*n* = 3). When there is only a weak stimulation, results are expressed as percentages of the endogenous agonist maximal stimulation at [ligand] = 1 *µ*M (*n* = 2). Results are not significant (ns) when there is <10% response at [ligand] = 1 *µ*M (*n* = 2).

| 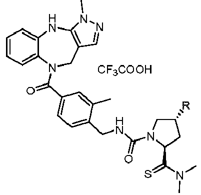 | | **OT-R n=3** | **OT-R n=3** |
|---|---|---|---|
| **Compound** | **R** | **EC₅₀ (nM)** | **Emax (%)** |
| Reference (not part of the invention) | -H | 45.1 | 96.7 |
| 1 | -F | 10.2 | 100 |
| 3 | 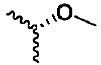 | 33 | |
| 4 | 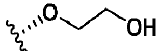 | 31.48 | 100 |
| 5 | 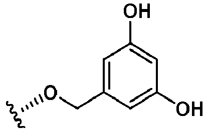 | 0.39 | 100 |

### Example 3 : V1A/OT selectivity assays

Comparison of efficacy as OT receptor agonist (measured as described in example 2), or as VIA receptor antagonist (measured as calcium signal, as follows), has been performed.

| 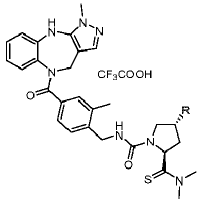 | | **OT-R (Ago) n=3** | **V_{1A}-R (Antago) n=3** | **Ratio V1A/OT n=3** |
|---|---|---|---|---|
| **Ref** | R | EC₅₀ (nM) | IC₅₀ (nM) | |
| **Reference** | -H | 45.1 | 346 | 7.7 |
| **23** | **-OCH₂CH(CH₃)₂** | 32.9 | 1007 | 31.5 |
| **1** | -F | 10.2 | 250 | 24 |

## Claims

1. A compound of following formula (I): 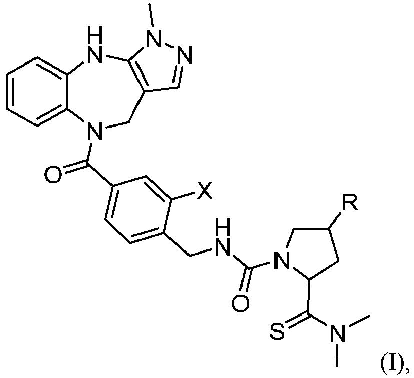 wherein:
X is chosen from -H, halogens, -(C₁-C₆)-alkyl groups, -O-(C₁-C₆)-alkyl groups and -O-CF₃, in particular -Me or -Cl, more particularly -Me;
R is chosen from the group comprising:
- -(C₁-C₆)-alkyl groups, in particular -Me;
- -O-(C₁-C₄)-alkyl groups, in particular -OMe;
- -S-(C₁-C₄)-alkyl groups, in particular -SMe;
- -O-(C₂-C₄)-alkyl-OH groups, in particular -O-(CH₂)ᵢ-OH, with i ranging from 2 to 4, i being more particularly 2;
- -O(-(C₂-C₄)-alkyl)ₙ-OH groups, in particular -O(-CH₂-CH₂)ₙ-OH, with n ranging from 2 to 4, n being more particularly 2;
- -O-(C₂-C₄)-alkyl-O-(C₂-C₄)-alkyl groups, in particular -O-(CH₂)ᵢ-OMe, with i ranging from 2 to 4, i being more particularly 2;
- -O(-(C₂-C₄)-alkyl)ₘ-(C₂-C₄)-alkyl groups, in particular -O(-CH₂-CH₂)ₘ-OMe or 2;
- -O-(C₁-C₄)-alkyl-Ar, in particular -O-(CH₂)ⱼ-Ar, with j ranging from 1 to 4, j being more particularly 1, and Ar being an aryl group being optionally substituted by at least one R" group, in particular at least one -OH group, said -O-(C₁-C₄)-alkyl-Ar group being for example of the following formula: 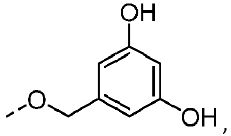 R" being chosen from the group comprising:
∘ -OH;
∘ a -O-(C₁-C₆)-alkyl or -O-cyclo-(C₃-C₆)-alkyl, in particular a -O-cyclopropyl;
∘ a -(C₁-C₆)-alkyl or -cyclo-(C₃-C₆)-alkyl, in particular a cyclopropyl;
∘ -(C₁-C₆)-alkyl-W groups, in particular -(CH₂)ₖ-W groups, said groups being optionally further substituted by at least a -W' group, with k ranging from 1 to 6, k being more particularly 1 or 2, and W and W' being ORₐ, with Rₐ representing -H or -(C₁-C₄)-alkyl, in particular H or Me, or a PEG group, in particular of following formula -(CH₂-CH₂-O)ₗ-H, with 1 ranging from 1 to 10, in particular from 2 to 4, for example 2; or W and W' being NR_{b}H, with R_{b} representing -H, a -(C₁-C₄)-alkyl group, or a -(C₁-C₄)-alkyl-Ar', with Ar' being an aryl or heteroaryl group, in particular a furanyl;
∘ -(C₁-C₆)-alkyl-Y-Z groups, in particular -(CH₂)ₗ-Y-Z, with 1 ranging from 1 to 6,1 being more particularly 1 or 2, Y being -O-, -NH- or being absent, and Z being chosen from saturated heterocyclic groups, in particular morpholinyl, tetrahydropyranyl, imidazolidinyl, said saturated heterocyclic groups being optionally substituted by at least one =O group, representing for example imidazolidine-2,4-dione; and heteroaryl groups, in particular tetrazolyl;
∘ An aryl or heteroaryl group, in particular a pyrazolyl, said group being optionally substituted by at least one R_{c} group chosen from -(C₁-C₆)-alkyl and -NH₂ groups;
∘ a -(C₀-C₆)-alkyl-C(=O)NH₂ or -(C₀-C₆)-alkyl-S(=O)₂NH₂ group, in particular a -C(=O)NH₂ or -(CH₂)₂-S(=O)₂NH₂ group;
- Halogens, in particular -F;
- -CF₃;
or R is -F and its geminal hydrogen is replaced by -F, R forming with the carbon atom C to which it is bound and its -F replaced geminal hydrogen a CF₂ residue;
or an enantiomer, diastereoisomer, and/or a pharmaceutically acceptable salt or solvate thereof.

2. A compound according to claim 1, of following formula (I): 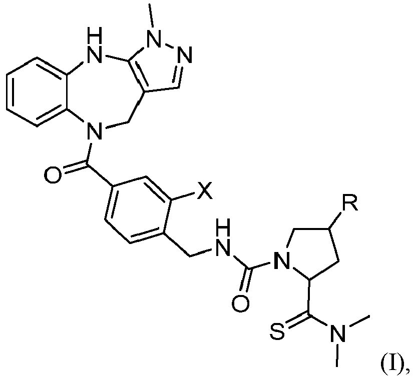 wherein:
X is -Me or -Cl, in particular -Me;
R is chosen from the group comprising:
- -O-(C₁-C₄)-alkyl groups, in particular -OMe;
- -O-(C₂-C₄)-alkyl-OH groups, in particular -O-(CH₂)ᵢ-OH, with i ranging from 2 to 4, i being more particularly 2;
- -O(-(C₂-C₄)-alkyl)ₙ-OH groups, in particular -O(-CH₂-CH₂)ₙ-OH, with n ranging from 2 to 4, n being more particularly 2;
- -O-(C₂-C₄)-alkyl-O-(C₂-C₄)-alkyl groups, in particular -O-(CH₂)ᵢ-OMe, with i ranging from 2 to 4, i being more particularly 2;
- -O(-(C₂-C₄)-alkyl)ₘ-(C₂-C₄)-alkyl groups, in particular -O(-CH₂-CH₂)ₘ-OMe or -O(-CH₂-CH₂)ₘ-OEt, with m ranging from 2 to 4, m being more particularly 2;
- -O-(C₁-C₄)-alkyl-Ar, in particular -O-(CH₂)ⱼ-Ar, with j ranging from 1 to 4, j being more particularly 1, and Ar being an aryl group being optionally substituted by at least one R" group as defined in claim 1, in particular at least one -OH group, said - O-(C₁-C₄)-alkyl-Ar group being for example of the following formula: 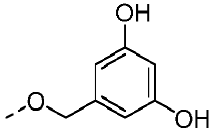
- Halogens, in particular -F.

3. A compound according to claim 1, of following formula (I): 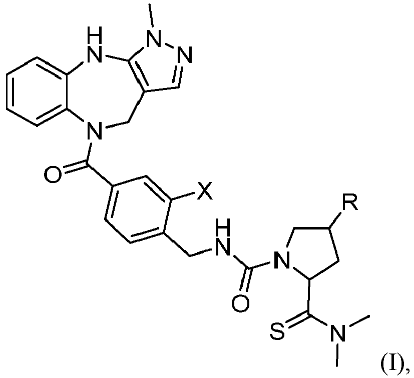 wherein:
X is -Me or -Cl, in particular -Me;
R is chosen from
-O-(C₁-C₄)-alkyl-Ar groups, in particular -O-(CH₂)ⱼ-Ar, with j ranging from 1 to 4, j being more particularly 1, and Ar being an aryl group being optionally substituted by at least one R" group as defined in claim 1, in particular at least one -OH group, said
-O-(C₁-C₄)-alkyl-Ar group being for example of the following formula:

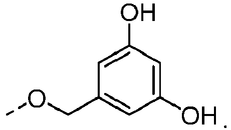

4. A compound according to claim 1, of one of the following formulae: 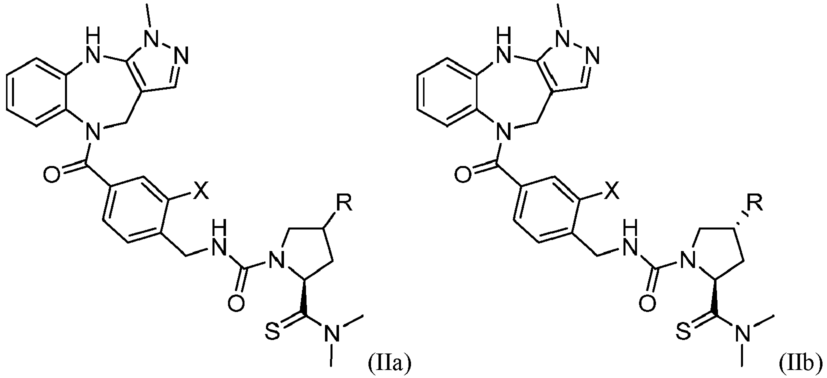 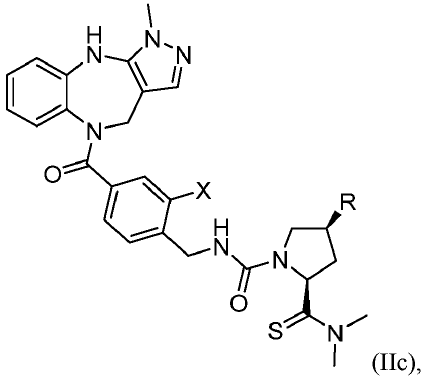 wherein X and R are as defined in claim 1.

5. A compound according to anyone of claims 1 to 4, wherein X is Me.

6. A compound according to anyone of claims 1 to 4, wherein X is Cl.

7. A compound according to anyone of claims 1 to 6, wherein R is chosen from:

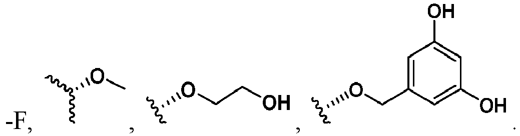

8. A pharmaceutical, dermatologic or cosmetic composition comprising a compound according to anyone of claims 1 to 7 in admixture with at least one pharmaceutical acceptable excipient.

9. A compound according to anyone of claims 1 to 7 for use in the prevention and/or treatment of a disease, condition or disorder, which is autism, autism spectrum disorders, in particular for the treatment of social interaction impairments in autism spectrum disorders, pain, drug and/or alcohol addiction, depression, post-traumatic stress, anxiety, eating disorders, notably anorexia, bulimia and Prader-Willi syndrome, schizophrenia, neurodevelopmental disorders, social interaction disorders, borderline behaviour, ageing of the skin, muscles, bones and any disease for which oxytocin is known to have a beneficial effect.

10. A composition comprising at least one compound according to anyone of claims 1 to 7 and a diuretic, for example urea or its salt, or a V2r antagonist as a combination product for simultaneous, separate or spread therapeutic use in the prevention and/or treatment of a disease, condition or disorder, which is autism, autism spectrum disorders, in particular for the treatment of social interaction impairments in autism spectrum disorders, pain, drug and/or alcohol addiction, depression, post-traumatic stress, anxiety, eating disorders, notably anorexia, bulimia and Prader-Willi syndrome, schizophrenia, neurodevelopmental disorders, social interaction disorders, borderline behaviour, ageing of the skin, muscles, bones and any disease for which oxytocin is known to have a beneficial effect.
